(19)

Europäisches
Patentamt
European
Patent Office
Office européen
des brevets

(11) **EP 1 858 492 B1**

(12) **EUROPEAN PATENT SPECIFICATION**

(45) Date of publication and mention
of the grant of the patent:
**12.11.2008 Bulletin 2008/46**

(21) Application number: **06734068.7**

(22) Date of filing: **27.01.2006**

(51) Int Cl.:
***A61K 9/28*** (2006.01)

(86) International application number:
**PCT/US2006/003273**

(87) International publication number:
**WO 2006/081569 (03.08.2006 Gazette 2006/31)**

(54) **ORAL OSMOTIC DOSAGE FORM HAVING A HIGH FLUX MEMBRANE**

ORALE OSMOTISCHE DARREICHUNGSFORM MIT MEMBRAN MIT HOHER FLUSSDICHTE

FORME GALENIQUE OSMOTIQUE ORALE COMPRENANT UNE MEMBRANE A FLUX ELEVE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **27.01.2005 US 647864 P**

(43) Date of publication of application:
**28.11.2007 Bulletin 2007/48**

(73) Proprietor: **ALZA Corporation
Mountain View, CA 94039-7210 (US)**

(72) Inventors:
• **EDGREN, David, E.
Los Altos, CA 94022 (US)**

• **BHATTI, Gurdish, K.
Fremont, CA 94539 (US)**
• **LI, Shu
Union City, CA 94587 (US)**
• **SKLUZACEK, Robert
Newark, CA 94560 (US)**

(74) Representative: **Williams, Paul Edwin et al
Ablett & Stebbing
Caparo House
101-103 Baker Street
London W1U 6FQ (GB)**

(56) References cited:
**US-A- 5 413 572        US-A- 6 077 538
US-A1- 2004 115 262      US-B1- 6 368 626**

**Description**

FIELD OF THE INVENTION

The invention relates to oral osmotic dosage forms and methods wherein a semi-permeable membrane of the oral osmotic dosage form includes from 23 wt% to 50 wt% of a flux enhancer and from 0.01 wt% to 5 wt% of antioxidant, based on the total dry weight of the semi-permeable membrane. Such compositions provide high flux membranes with improved mechanical and transport stability during storage on the shelf.

BACKGROUND

One type of oral dosage from is an oral osmotic dosage form. Osmotic dosage forms, in general, utilize osmotic pressure to generate a driving force for imbibing fluid into a compartment formed, at least in part, by a semi-permeable membrane that permits free diffusion of fluid but not drug. An advantage to osmotic systems is that their operation is pH-independent and, thus, continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release, 35:1-21 (1995). Osmotic dosage forms are also described in detail in the following U.S. Patents Nos. 3,845,770; 3,916,899; 3,995,631; 4,008,719; 4,111,202; 4,160,020; 4,327,725; 4,519,801; 4,578,075; 4,681,583; 5,019,397; and 5,156,850.

One characteristic of such oral osmotic dosage forms is that a period of time, known as the start up time, is required before the osmotic dosage form begins to dispense drug from within the dosage form. This is due to the time that is required for water in the environment of use to be imbibed through the semi-permeable membrane and induce the dispensing process.

In certain instances, for example when faster dispensing times are desired, the start up time may be undesirably long. This may be addressed by adding additional structures to the dosage form, such as immediate release overcoats, but such additional structures raise the cost of the dosage form. Another way in which shorter startup times may be provided is to increase the amount of water flux through the semi-permeable membrane. This may be accomplished through the incorporation of flux enhancers in the semi-permeable membrane. In general, greater amounts of flux enhancers present within the semi-permeable membrane result in increases in water flux across the semi-permeable membrane.

US Patent No. 2004/115262 describes an oral osmotic dosage from with a semipermeable membrane consisting of 70% cellulose acetate and 30 % Lutrol F68.

However, at higher levels of flux enhancer incorporation, stability problems have been noted with oral osmotic dosage forms. In particular, release rates have been found to vary significantly over time for oral osmotic dosage forms having higher levels of flux enhancer incorporation, as compared to oral osmotic dosage forms having lower levels of flux enhancer incorporation. Additionally, mechanical properties of the semi-permeable membrane that are required for the delivery system to function properly and to be sufficiently durable to allow handling and packaging have been found to degrade over time for dosage forms having semi-permeable membranes with higher levels of flux enhancer incorporation as compared to oral dosage forms having membranes with lower levels of flux enhancer incorporation.

The reasons for these stability problems have not been known in the art. Therefore, dosage forms and methods that address such problems, while providing increased water flux and improved tensile properties, are needed.

SUMMARY OF THE INVENTION

In an aspect, the invention relates to an oral osmotic dosage form comprising: a core that comprises a drug layer; a semi-permeable membrane surrounding the core; and an exit port through the semi-permeable membrane; wherein the semi-permeable membrane comprises from 23 wt% to 50 wt% of a flux enhancer and from 0.01 wt% to 5 wt% of antioxidant, based on the total dry weight of the semi-permeable membrane.

In another aspect, the invention relates to a method of making an oral osmotic dosage form comprising: providing a semi-permeable membrane that comprises from 23 wt% to 50 wt% of a flux enhancer and from 0.01 wt% to 5 wt% of antioxidant, based on the total dry weight of the semi-permeable membrane; coating the semi-permeable membrane on a core that comprises a drug layer and forming an exit port through the semipermeable membrane.

BRIEF DESCRIPTION OF THE DRAWINGS

Figure 1 shows plots of two year stability of oral osmotic dosage forms stored at room temperature.

Figure 2 shows plots of two year stability of oral osmotic dosage forms stored at 40 degrees centigrade.

Figure 3 shows plots of two year stability of oral osmotic dosage forms stored at 50 degrees centigrade.

Figure 4 shows Arrhenious plots of two year release performance of oral osmotic dosage forms.

Figure 5 shows plots of tensile properties of rate controlling membranes of oral osmotic dosage forms after storage for one year at several temperatures.

Figure 6 shows results from comparison of actual versus theoretical BHT content within rate controlling membranes stored for two years at several temperatures.

Figure 7 shows plots of the effect of BHT on molecular weight and permeability of rate controlling membranes blended with a flux enhancer on oral osmotic dosage forms stored two years at several temperatures.

Figure 8 shows plots of the effect of BHT on molecular weight and permeability of rate controlling membranes blended with a different flux enhancer on oral osmotic dosage forms after storage for two years.

Figure 9 shows an elementary osmotic pump dosage form.

Figure 10 shows an oral osmotic dosage form comprising a push layer.

Figures 11 and 12 shows oral osmotic dosage forms comprising liquid drug formulations.

DETAILED DESCRIPTION OF THE PREFERRED EMBODIMENTS DEFINITIONS

"Antioxidant" means agents that inhibit oxidative degradation. Examples of antioxidants useful in the practice of this invention are presented elsewhere herein.

"BHT" means butylated hydroxytoluene.

"Coating" means to form a film on a surface.

"Core" means a portion of a dosage form that comprises a drug layer, and optionally additional layers.

"Drug layer" means that portion or those portions of a dosage form that comprises an active pharmaceutical ingredient.

"Exit port" means a hole or passageway formed through the semi-permeable membrane of an osmotic dosage form. Examples of exit ports and methods of making them that are useful in the practice of this invention are presented elsewhere herein.

"Flux enhancer" means a substance added to assist in increasing fluid (water, for example) permeability or flux through a semi-permeable membrane. In certain embodiments, the flux enhancer comprises a water soluble agent blended into the semi-permeable membrane.

"Oral" means orally administered, when used to describe a dosage form.

"Osmotic dosage form" means dosage forms that, in general, utilize osmotic pressure to generate a driving force for imbibing fluid into a compartment formed, at least in part, by a semipermeable membrane that permits free diffusion of fluid but not drug. Examples of osmotic dosage forms useful in the practice of this invention are presented elsewhere herein.

"EOP" means an osmotic dosage form comprising a rate controlling membrane with at least one delivery port which membrane encapsulates a single layer, water soluble drug core.

"Patient" means an animal, preferably a mammal, more preferably a human, in need of therapeutic intervention.

"Semi-permeable Membrane" or "Membrane" means a membrane that is permeable to the passage of an external fluid, such as water and/or biological fluids, but is substantially impermeable to the passage of components such as active pharmaceutical ingredients. Materials useful for forming the semi-permeable membrane are essentially nonerodible and are substantially insoluble in biological fluids during the life of a dosage form that comprises the semi-permeable membrane. Materials and methods for forming the semi-permeable membrane, and structures of osmotic dosage forms that comprise the semi-permeable membrane are disclosed elsewhere herein.

"High flux membrane" means a semi-permeable membrane that comprises a coating that comprises a blend of water insoluble and water-soluble components where the water-soluble components are present within the coating in a weight percent range of 23 to 50 percent, based on the total weight of the dry semi-permeable membrane.

"Shelf Life" or "Stability" means the time required for a dosage form stored at room temperature to undergo a 5% change in release rate compared to a similarly constructed dosage form tested at time zero. If over the period of testing, the release performance as measured by average release rate of the dosage form remains within a 5% change in release rate compared to a similarly constructed dosage form tested at time zero, it is said to be stable.

HIGH FLUX MEMBRANE STABILITY

The inventors have unexpectedly discovered that the reason for stability problems with oral osmotic dosage forms having high levels of flux enhancers is degradation of polymers that provide semi-permeable properties to the membrane. This result is surprising because flux enhancers have been incorporated into semi-permeable membranes at lower levels without inducing stability problems. The inventors have further discovered that these stability/shelf-life problems can be addressed by the addition of enhanced amounts of antioxidants to the semi-permeable membrane of which the oral osmotic dosage form is comprised. This solution to the problem has advantages because it is easy to implement.

The invention will now be described in more detail.

ORAL OSMOTIC DOSAGE FORMS

A variety of oral osmotic dosage forms are disclosed herein as being useful in the practice of this invention. This listing and description herein is not meant to be exhaustive, as a wide variety of oral osmotic dosage forms are known in the art. In certain embodiments, the present invention is meant to be applicable to those oral osmotic dosage forms comprising semi-permeable membranes that comprise flux enhancer in amounts of at least about 23 wt%, including those known in the art but not expressly disclosed herein.

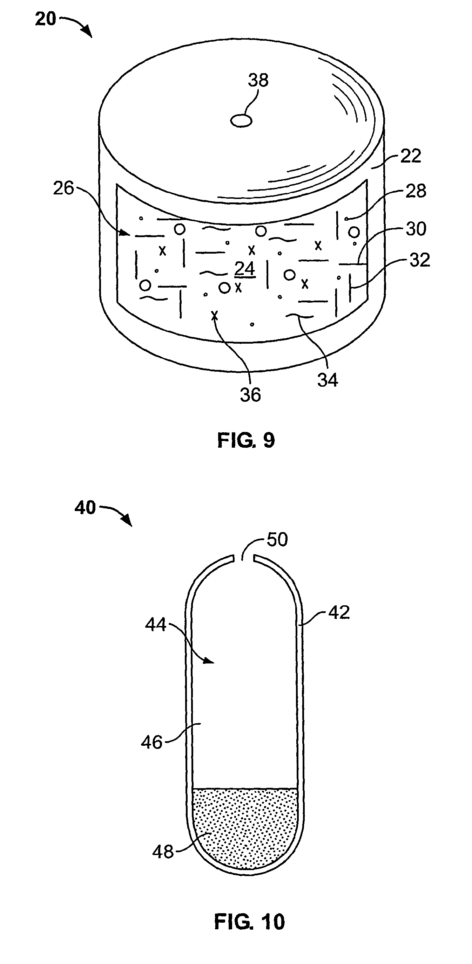

An exemplary dosage form, referred to in the art as an elementary osmotic pump dosage form, is shown in Fig. 9. Dosage form 20, shown in a cutaway view, is also referred to as an elementary osmotic pump, and is comprised of a semi-permeable membrane 22 that surrounds and encloses an internal compartment 24. The internal compartment contains a single component layer referred to herein as a drug layer 26, comprising an inventive substance 28 in an admixture with selected excipients. The excipients are adapted to provide an osmotic activity gradient for attracting fluid from an external environment through membrane 22 and for forming a deliverable complex formulation upon imbibition of fluid. The excipients may include a suitable suspending agent, also referred to herein as drug carrier 30, a binder 32, a lubricant 34, and an osmotically active agent referred to as an osmagent 36. Exemplary materials for each of these components are provided below.

Semi-permeable membrane 22 of the osmotic dosage form is permeable to the passage of an external fluid, such as water and biological fluids, but is substantially impermeable to the passage of drug present within the internal compartment. Materials useful for forming the membrane are essentially nonerodible and are substantially insoluble in biological fluids during the life of the dosage form. Materials for forming the membrane are disclosed elsewhere herein, and examples of membrane forming materials may be found in the art.

The exit orifice can be formed by mechanical drilling, laser drilling, eroding an erodible element, extracting, dissolving, bursting, or leaching a passageway across from the composite membrane. The exit orifice can be a pore formed by leaching sorbitol, lactose or the like from a membrane or layer as disclosed in U.S. Pat. No. 4,200,098. This patent discloses pores of controlled-size porosity formed by dissolving, extracting, or leaching a material from a membrane, such as sorbitol from cellulose acetate. A preferred form of laser drilling is the use of a pulsed laser that incrementally removes material from the composite membrane to the desired depth to form the exit orifice. The above processes may be used with any suitable oral osmotic dosage form according to the invention.

In operation, the osmotic gradient across membrane 22 due to the presence of osmotically-active agents causes fluid of the biological environment to be imbibed through the membrane, hydrating the drug layer, and forming a deliverable complex formulation (e.g., a solution, suspension, slurry or other flowable composition) within the internal compartment. The deliverable inventive substance formulation is released through an exit 38 as fluid continues to enter the internal compartment. Even as drug formulation is released from the dosage form, fluid continues to be drawn into the internal compartment, thereby driving continued release. In this manner, the inventive substance is released in a sustained and continuous manner over an extended time period.

Fig. 10 is a schematic illustration of another exemplary oral osmotic dosage form. Dosage forms of this type are described in detail in U.S. Patent Nos.: 4,612,008; 5,082,668; and 5,091,190. In brief, dosage form 40, shown in cross-section, has a semi-permeable membrane 42 defining an internal compartment 44. Internal compartment 44 contains a bilayered-compressed core having a drug layer 46 and a push layer 48. As will be described below, push layer 48 is a displacement composition that is positioned within the dosage form such that as the push layer expands during use, the materials forming the drug layer are expelled from the dosage form via one or more exit ports, such as exit port 50. The push layer can be positioned in contacting layered arrangement with the drug layer, as illustrated in Fig. 10, or can have one or more intervening layers separating the push layer and drug layer.

Drug layer 46 comprises an inventive substance an admixture with selected excipients, such as those discussed above with reference to Fig. 10. An exemplary dosage form can have a drug layer comprised of an inventive substance, a poly(ethylene oxide) as a carrier, sodium chloride as an osmagent, hydroxypropyl methylcellulose as a binder, and magnesium stearate as a lubricant.

Push layer 48 comprises osmotically active component(s), such as one or more polymers that imbibes an aqueous or biological fluid and swells, that are referred to in the art as an osmopolymer. Osmopolymers are swellable, hydrophilic polymers that interact with water and aqueous biological fluids and swell or expand to a high degree, typically exhibiting a 2-50 fold volume increase. The osmopolymer can be non-crosslinked or crosslinked, and in a preferred embodiment the osmopolymer is at least lightly crosslinked to create a polymer network that is too large and entangled to easily exit the dosage form during use. Examples of polymers that may be used as osmopolymers are provided in the references noted above that describe osmotic dosage forms in detail. A typical osmopolymer is a poly(alkylene oxide), such as poly(ethylene oxide), and a poly(alkali carboxymethylcellulose), where the alkali is sodium, potassium, or lithium. Additional excipients such as a binder, a lubricant, an antioxidant, and a colorant may also be included in the push layer. In use, as fluid is imbibed across the semi-permeable membrane, the osmopolymer(s) swell and push against the drug layer to cause release of the drug from the dosage form via the exit port(s).

The push layer can also include a component referred to as a binder, which is typically a cellulose or vinyl polymer, such as poly-n-vinylamide, poly-n-vinylacetamide, poly(vinyl pyrrolidone), poly-n-vinylcaprolactone, poly-n-vinyl-5-methyl-2-pyrrolidone, and the like. The push layer can also include a lubricant, such as sodium stearate or magnesium stearate, and an antioxidant to inhibit the oxidation of ingredients. Representative antioxidants include, but are not limited to, ascorbic acid, ascorbyl palmitate, butylated hydroxyanisole, a mixture of 2 and 3 tertiary-butyl-4-hydroxyanisole, and butylated hydroxytoluene.

An osmagent may also be incorporated into the drug layer and/or the push layer of the osmotic dosage form. Presence of the osmagent establishes an osmotic activity gradient across the semi-permeable membrane. Exemplary osmagents include salts, such as sodium chloride, potassium chloride, lithium chloride, etc. and sugars, such as raffinose, sucrose, glucose, lactose, and carbohydrates.

The oral osmotic dosage form can optionally include an overcoat (not shown) for color coding the dosage forms according to dose or for providing an immediate release of a drug or another drug.

In use, water flows across the membrane and into the push layer and the drug layer. The push layer imbibes fluid and begins to swell and, consequently, pushes on drug layer 44 causing the material in the layer to be expelled through the exit orifice and into the gastrointestinal tract. Push layer 48 is designed to imbibe fluid and continue swelling, thus continually expelling the inventive substance from the drug layer.

In an embodiment, the present invention provides a liquid drug formulation for use with oral osmotic dosage forms. Oral osmotic dosage forms for delivering liquid drug formulations and methods of using them are known in the art, for example, as described and claimed in the following U.S. Patents owned by ALZA corporation: 4,111,201; 5,324,280; 6,174,547; 6,419,952; and 6,551,613.

Exemplary liquid carriers for the present invention include lipophilic solvents (e.g., oils and lipids), surfactants, and hydrophilic solvents. Exemplary lipophilic solvents, for example, include, but are not limited to, Capmul PG-8, Caprol MPGO, Capryol 90, Plurol Oleique CC 497, Capmul MCM, Labrafac PG, N-Decyl Alcohol, Caprol 10G100, Oleic Acid, and Vitamin E. Exemplary surfactants for example, include, but are not limited to, Vitamin E TPGS, Cremophor EL-P, Labrasol, Tween 20, and Cremophor RH40. Exemplary hydrophilic solvents for example, include, but are not limited to, Isosorbide Dimethyl Ether, polyethylene glycol 200 (PEG 200), polyethylene glycol 300 (PEG 300), polyethylene glycol 400 (PEG 400), polyethylene glycol 600 (PEG 600), methoxypolyethylene glycol 350 (MPEG 350), methoxypolyethylene glycol 550 (MPEG 550), Transcutol HP, and Propylene Glycol (PG), and combinations of these solvents.

The skilled practitioner will understand that any formulation comprising a sufficient dosage of inventive substances solubilized in a liquid carrier suitable for administration to a subject and for use in an osmotic device can be used in the present invention.

The liquid formulation according to the present invention can also comprise, for example, additional excipients such as an antioxidant, permeation enhancer and the like. Antioxidants can be provided to slow or effectively stop the rate of any autoxidizable material present in the capsule. Antioxidants are known to the prior art in U.S. Pat. Nos.: 2,707,154; 3,573,936; 3,637,772; 4,038,434; 4,186,465; and 4,559,237.

In certain embodiments, the inventive substances are administered as a self-emulsifying formulation. Like the other liquid carriers, the surfactant functions to prevent aggregation, reduce interfacial tension between constituents, enhance the free-flow of constituents, and lessen the incidence of constituent retention in the dosage form. The emulsion formulation of this invention comprises a surfactant that imparts emulsification.

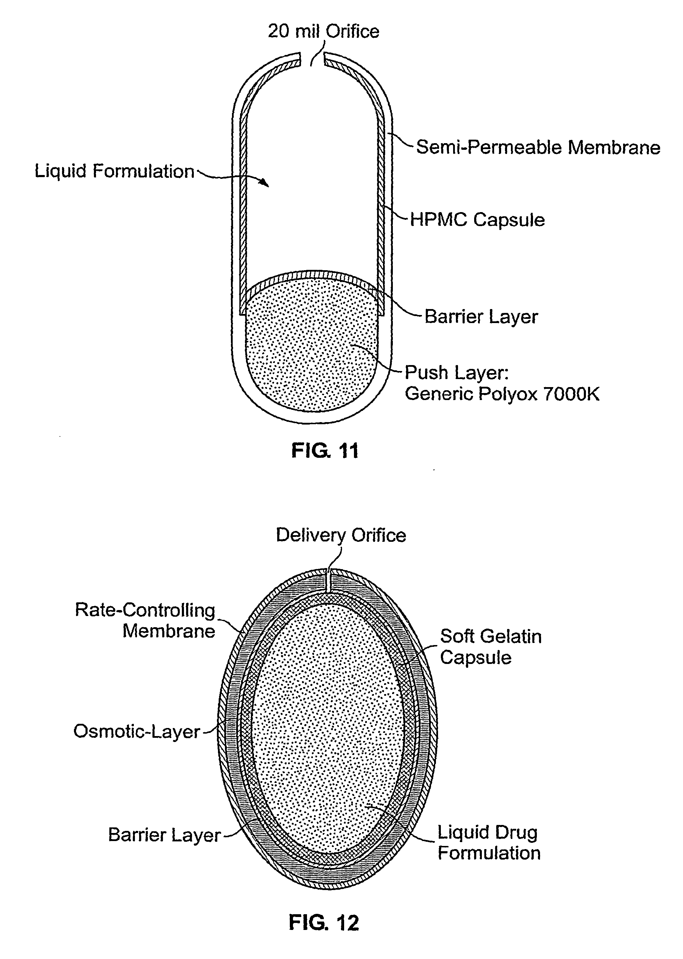

The osmotic dosage forms of the present invention can additionally possess two distinct forms, a soft capsule form (shown in Fig. 12) and a hard capsule form (shown in Fig. 11). Methods of forming hard cap dosage forms are described in U.S. Pat. Nos.: 6,174,547; 6,419,952; and 6,596,314.

The semipermeable membrane surrounds and forms a compartment containing a plurality of layers within the soft or hard capsule osmotic dosage form, one of which is an expandable layer which in some embodiments, can contain osmotic agents. The expandable layer comprises in one embodiment a hydroactivated composition that swells in the presence of water, such as that present in gastric fluids. Conveniently, it can comprise an osmotic composition comprising an osmotic solute that exhibits an osmotic pressure gradient across the semipermeable layer against an external fluid present in the environment of use. In another embodiment, the hydro-activated layer comprises a hydrogel that imbibes and/or absorbs fluid into the layer through the outer semipermeable membrane. The semipermeable membrane is non-toxic. It maintains its physical and chemical integrity during operation and it is essentially free of interaction with the expandable layer. The semi-permeable membrane is discussed further elsewhere herein.

The expandable layer in one preferred embodiment of the soft or hard capsule osmotic dosage form comprises a hydroactive layer comprising a hydrophilic polymer, also known as osmopolymers. The osmopolymers exhibit fluid imbibition properties. The osmopolymers are swellable, hydrophilic polymers, which osmopolymers interact with water and biological aqueous fluids and swell or expand to an equilibrium state. The osmopolymers exhibit the ability to swell in water and biological fluids and retain a significant portion of the imbibed fluid within the polymer structure. The osmopolymers swell or expand to a very high degree, usually exhibiting a 2 to 50 fold volume increase. The osmopolymers can be noncross-linked or cross-linked. The swellable, hydrophilic polymers are in one embodiment lightly cross-linked, such crosslinks being formed by covalent or ionic bonds or residue crystalline regions after swelling. The osmopolymers can be of plant, animal or synthetic origin.

In certain embodiments, the soft or hard capsule osmotic dosage form dosage forms further can comprise a barrier layer. The barrier layer in certain embodiments is deformable under the pressure exerted by the expandable layer and will be impermeable (or less permeable) to fluids and materials that can be present in the expandable layer, the liquid active agent formulation and in the environment of use, during delivery of the active agent formulation.

The various layers forming the barrier layer, expandable layer and semipermeable layer can be applied by conventional coating methods such as described in U.S. Patent No. 5,324,280. While the barrier layer, expandable layer and semipermeable membrane have been illustrated and described for convenience as single layers, each of those layers can be composites of several layers.

Materials and methods disclosed herein as being useful in the making of one type of oral osmotic dosage form may be usefully applied in the making of another type of oral osmotic dosage form. Cross-application of such materials and methods is conventionally known.

HIGH FLUX MEMBRANES

The osmotic devices of the present invention comprise a semipermeable membrane permeable to the passage of exterior biological fluid and substantially impermeable to the passage of drug formulation. The selectively permeable compositions used for forming the membrane are essentially non-erodible and they are insoluble in biological fluids during the functional life of the osmotic system. The semipermeable membrane comprises a composition that does not adversely affect the host, the drug formulation, an osmopolymer, osmagent and the like. As mentioned above, in certain circumstances, high fluid flux through the semi-permeable membrane is desirable (to shorten start up times, for instance). High flux rate controlling membranes are those that comprise a membrane blend consisting of high fraction of water soluble flux enhancer within the water insoluble polymeric membrane. In such circumstances, increased levels of flux enhancers are incorporated into the semi-permeable membranes.

In a conventional oral osmotic dosage form, a typical flux semi-permeable membrane may comprise from 77 wt% to 100 wt% semi-permeable polymer, 0 wt% to 23 wt % flux enhancer, and < 0.0023% antioxidant. In an embodiment, an inventive semi-permeable membrane may comprise from 50 wt% to 77 wt% semi-permeable polymer, 23 wt% to 50 wt% flux enhancer, and 0.01 wt% to 5.0 wt% antioxidant. All weight percents of the components of the semi-permeable membrane are based on the total dry weight of the semi-permeable membrane. As can be seen by inspection, the inventive semi-permeable membranes comprise much higher levels of flux enhancers and antioxidants as compared to a more typical low flux semi-permeable membrane of the prior art.

In certain embodiments, the high flux membrane may be prepared by dissolving with stirring the semi-permeable polymer, flux enhancer, and an antioxidant(s) in a suitable solvent system. Optionally, the solution can be warmed while stirring to promote complete and rapid dissolution. A preferred semipermeable polymer is cellulose acetate as manufactured and supplied by Eastman Chemical Company, Kingsport, TN. This polymer is supplied without an antioxidant. Preferred flux enhancers are Lutrol® F127 or Lutrol® F68 as manufactured and supplied by the BASF Corporation, Mount Olive, NJ. Lutrol is generally supplied by the manufacturer with a nominal concentration of 100 ppm BHT as a stabilizer. A preferred antioxidant of the present invention is BHT supplied by Uniroyal Chemical, Geismar, La. A preferred solvent system is acetone. Alternately, a solvent system consisting of acetone blended with up to about 3 percent water is preferred.

Representative semi-permeable polymers useful in forming the semi-permeable membrane comprise semi-permeable homopolymers, semi-permeable copolymers, and the like. In one presently preferred embodiment, the compositions can comprise cellulose esters, cellulose ethers, and cellulose ester-ethers. The cellulosic polymers typically have a degree of substitution, "D.S.", on their anhydroglucose unit from greater than 0 up to 3 inclusive. By degree of substitution is meant the average number of hydroxyl groups originally present on the anhydroglucose unit that are replaced by a substituting group, or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkylsulfamate, semipermeable polymer forming groups, and the like. The semipermeable compositions typically include a member selected from the group consisting of cellulose acylate, cellulose diacylate, cellulose triacylate, cellulose triacetate, cellulose acetate, cellulose diacetate, mono-, di- and tri-cellulose alkanylates, mono-, di-, and tri-alkenylates, mono-, di-, and tri-aroylates, and the like. Exemplary polymers can include, for example, cellulose acetate have a D.S. of 1.8 to 2.3 and an acetyl content of 32 to 39.9%; cellulose diacetate having a D.S. of 1 to 2 and an acetyl content of 21 to 35%, cellulose triacetate having a D.S. of 2 to 3 and an acetyl content of 34 to 44.8%, and the like. More specific cellulosic polymers include cellulose propionate having a D.S. of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a D.S. of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a D.S. of 2.6 to 3 such as cellulose trivalerate, cellulose trilamate, cellulose tripalmitate, cellulose trioctanoate, and cellulose tripropionate; cellulose diesters having a D.S. of 2.2 to 2.6 such as cellulose disuccinate, cellulose dipalmitate, cellulose dioctanoate, cellulose dicarpylate, and the like; mixed cellulose esters such as cellulose acetate valerate, cellulose acetate succinate, cellulose propionate succinate, cellulose acetate octanoate, cellulose valerate palmitate, cellulose acetate heptonate, and the like. Semipermeable polymers are known in U.S. Pat. No. 4,077,407 and they can be synthesized by procedures described in Encyclopedia of Polymer Science and Technology, Vol. 3, pages 325 to 354, 1964, published by Interscience Publishers, Inc., New York. Additional semipermeable polymers for forming the semipermeable membrane can comprise, for example, cellulose acetaldehyde dimethyl acetate; cellulose acetate ethylcarbamate; cellulose acetate methylcarbamate; cellulose dimethylaminoacetate; semipermeable polyamide; semipermeable polyurethanes; semipermeable sulfonated polystyrenes; cross-linked selectively semipermeable polymers formed by the coprecipitation of a polyanion and a polycation as disclosed in U.S. Pat. Nos. 3,173,876; 3,276,586; 3,541,005; 3,541,006; and 3,546,142; semipermeable polymers as disclosed in U.S. Pat. No. 3,133,132; semipermeable polystyrene derivatives; semipermeable poly (sodium styrenesulfonate); semipermeable poly (vinylbenzyltremethylammonium chloride); semipermeable polymers, exhibiting a fluid permeability of 10 exp-5 to 10 exp-2 (cm mil/atm hr) expressed as per atmosphere of hydrostatic or osmotic pressure differences across a semipermeable membrane. The polymers are known to the art in U.S. Pat. Nos. 3,845,770; 3,916,899; and 4,160,020; and in Handbook of Common Polymers, by Scott, J. R., and Roff, W. J., 1971, published by CRC Press, Cleveland. Ohio.

The semi-permeable membrane can also comprise a flux enhancer. Flux enhancers are often essentially hydrophilic. In an embodiment, the amount of flux enhancer in the semi-permeable membrane is at least 23 wt%, preferably from 23 wt% to 50 wt%, more preferably from 25 wt% to 45 wt%, and even more preferably from 30 wt% to 40 wt%, based on the total dry weight of the semi-permeable membrane.

Types of enhancers encompassed by this invention are those with molecular structures that include one or more polyethylene oxide or polyethylene glycol moieties with repeat monomer repeat units of ethylene oxide or ethylene glycol. The average number of repeat units per flux enhancer ranges from 3 to 2,500, with a preferred range of 20 to 400, and with an especially preferred range of 150 to 250. Such polymers are selected from the group including polyethylene glycol, polyethylene oxide, di-block co-polymers of polyoxyethylene-polyoxypropylene, tri-block co-polymers of polyoxyethylene-polyoxypropylene- polyoxyethylene, tri-block co-polymers of polyoxypropylene-polyoxyethylene-polyoxypropylene, tetrafunctional block copolymers derived from the sequential addition of propylene oxide and then ethylene oxide to ethylene diamine, tetrafunctional block copolymers derived from the sequential addition of ethylene oxide and then propylene oxide to ethylene diamine, polyethylene glycol and polyethylene oxide esters of fatty acids such as polyethylene glycol myristate, polyethylene glycol palmitate, polyethylene glycol oleate, polyethylene glycol stearate, polyethylene glycol oleate, polyethylene glycol linolate, polyoxyl dimyristate, polyoxyl dipalmitate, polyoxyl oleate, polyoxyl distearate, polyoxyl dioleate, polyoxyl distearate, glyceride esters of ethylene oxide such as polyoxyethylene castor oil, polyoxyethylene of hydrogenated castor oil, polyoxyethylene alkyl ethers, polyethylene glycol monocetyl ether, polyethylene glycol cetostearyl ether, polyethylene glycol lauryl ether, polyethylene glycol oleyl ether, polyethylene glycol stearyl ether, ethoxylated fatty alcohol, polyoxyethylene sorbitan fatty acid esters such as polyoxyethylene sorbitan monolaurate, polyoxyethylene sorbitan monopalmitate, polyoxyethylene sorbitan monostearate, polyoxyethylene sorbitan tristearate, polyoxyethylene sorbitan monooleate, polyoxyethylene sorbitan trioleate, polyoxyethylene sorbitan monoisostearate, polyoxyethylene sorbitan septaoleate, and polyoxyethylene isohexdecyl ether. Other flux enhancers include polyoxyethylene propylene glycol myristate, polyoxyethylene propylene glycol palmitate, polyoxyethylene propylene glycol oleate, polyoxyethylene propylene glycol linolate, polyethylene glycol caprylic/capric glycerides, polyethylene glycol hydroxymyristate, polyethylene glycol hydroxypalmitate, polyethylene glycol hydroxyoleate, polyethylene glycol hydroxylinoate, polyethylene glycol hydroxy stearate, polyethylene glycol hydroxy oleate, polyethylene glycol hydroxymyristate, polyethylene glycol hydroxypalmitate, polyethylene glycol lanolin, polyethylene glycol lanolin alcohol, polypropylene glycol polyethylene glycol lanolin, pegylated nut oils, such as polyethylene glycol peanut oil, polyethylene glycol almond oil, polyethylene glycol palm kernal oil, polyethylene glycol coconut oil pegylated grain oils such as pegylated corn oils, pegylated wheat germ oils, pegylated seed oils, such as pegylated cottonseed oil, pegylated safflower oils, pegylated sesame oils, pegylated fish oils such as pegylated shark liver oil, pegylated menhanden oils, pegylated cod oils, pegylated shark liver oils, pegylated olive oils, and the like. A more complete listing of pegylated compounds useful in the present invention can be found in McCutheon's Detergents and Emulsifiers, International Edition, 1979 Annual and BASF Pluronic and Tetronic Surfactants (1999).

Other materials that can be used to form the semipermeable membrane for imparting flexibility and elongation properties to the membrane, for making the membrane less-to-nonbrittle and to render tear strength, include, for example, phthalate plasticizers such as dibenzyl phthalate, dihexyl phthalate, butyl octyl phthalate, straight chain phthalates of six to eleven carbons, di-isononyl phthalte, di-isodecyl phthalate, and the like. The plasticizers include nonphthalates such as monoacetin, diacetin, triacetin, low molecular weight polyethylene glycol such as PEG 400, esters of citric acid including members selected from the group comprising trimethyl citrate, acetyl trimethyl citrate, triethyl citrate, acetyl triethyl citrate, tributyl citrate, acetyl tributyl citrate, tri-n-hexyl citrate, acetyl tri-n-hexyl citrate, butryl trihexyl citrate, tricyclohexyl citrate, acetyl tri-n-(hexyl/ocyl/decyl) citrate, acetyl tri-n-(octyl/decyl) citrate, acetyl tri-n-(decyl/dodecyl) citrate, tristearyl citrate, monostearyl citrate, and the like, dioctyl azelate, epoxidized tallate, tri-isoctyl trimellitate, tri-isononyl trimellitate, sucrose acetate isobutyrate, epoxidized soybean oil, ethylhexyl acetate, di(2-ethylhexyl) adipate, and the like. The amount of plasticizer in a membrane when incorporated therein is about 0.01 % to 20% weight, or higher.

The semi-permeable membrane solution may be spray coated onto the cores in a pan or column coater in a current of warm air until a sufficient thickness of coating is accumulated on each tablet. Preferred coating thicknesses are 3 to 12 mils, with a more preferred range of 4 to 6 mils. The resulting systems are drilled with one or more delivery ports and then dried in a forced air oven to remove residual coating solvent. Alternatively, the semi-permeable membrane may be laminated onto the core. Additional methods for forming semi-permeable membranes from the materials disclosed herein in the amounts disclosed herein may be adapted by one of skill in the art from the literature.

ANTIOXIDANTS

As discussed above, elevated levels of flux enhancers provide elevated flux levels, but also negatively impact stability of oral osmotic dosage forms during storage on the shelf. These stability problems are exemplified in the Examples below. Data in the Examples shows that such stability problems can be addressed by incorporating increased levels of antioxidants within the semi-permeable membrane. Inclusion of increased concentration of antioxidants provides for improved stability of the semi-permeable membrane polymer molecular weight during storage, as shown in the Examples below. The semi-permeable membrane polymer molecular weight must be stable in order for mechanical properties of the membrane to be stable.

The need for such increased levels of antioxidants in the semi-permeable membrane is unexpected because flux enhancers, at levels up to 17 wt% (based on total dry semi-permeable membrane weight) have been included in semi-permeable membranes in conventionally available and acceptably stable oral osmotic dosage forms. Moreover, conventional semipermeable membranes are commonly formulated with cellulose acetates. It is non-obvious that the stability of these cellulose acetate membranes would benefit by formulating them with antioxidant because these polymers are routinely supplied commercially devoid of antioxidants. Further, certain flux enhancers used in semi-permeable membranes, such as Lutrol ® F68 or Lutrol® F127, are supplied by the manufacturer pre-formulated with antioxidants. However, as can be seen in the Examples, when increased amounts of flux enhancers such as Lutrol® are incorporated into the semi-permeable membranes the amount of pre-formulated antioxidants is insufficient to prevent stability problems. The inventors have unexpectedly discovered that there is a threshold level of flux enhancer incorporation beyond which increased proportions of antioxidants are required to improve stability of the semi-permeable membrane, and consequently the oral osmotic dosage form. In an embodiment, when the semi-permeable membrane comprises from 23 wt% to 50 wt% of a flux enhancer, the semi-permeable membrane also comprises from 0.01 % to 5 wt% of antioxidant, based on the total dry weight of the semi-permeable membrane.

In an embodiment, an additional amount of antioxidant is added to the semi-permeable membrane such that the concentration of antioxidant relative to the total weight of the dry semi-permeable membrane is raised from a level of 0.002-0.005 weight percent to a level of 0.014-0.03 weight percent. In an embodiment, the semi-permeable membrane comprises from 0.01 wt% to 5 wt%, preferably from 0.01 wt% to 3 wt%, more preferably from 0.01 wt % to 1 wt %, and even more preferably from 0.014 wt% to 0.05 wt% of antioxidant, based on the total dry weight of the semi-permeable membrane. In an embodiment, the additional amount of antioxidant added within the dry membrane the level of antioxidant relative to the weight of the flux enhancer present within the membrane may be raised from 100 parts per million (ppm) as supplied by the manufacturer of the flux enhancer to the level of 300 ppm or more, preferably to 400 ppm or more, more preferably to 500 ppm or more, and even more preferably to 600 ppm or more.

Antioxidants useful in the present invention comprise butylated hydroxyanisol, butylated hydroxytoluene, butylated hydroxymethylphenol, teriary butylhydroquinone, tocopherols, phospholipids, lecithin, editic acid, ascorbic acid, isoascorbic acid, fumaric acid, malic acid, ascorbic acid palmitate, gallic acid, propyl gallate, sodium ascorbate, sodium sulfite, sodium metabisulfite, sodium bisulfite, sodium thiosulfate, sodium formaldehyde sulfoxylate, sulfur dioxide, thioglycerol, thioglycolic acid, cysteine hydrochloride, acetylcysteine, ascorbyl palmitate, hydroquinone, nordihydroguaiaretic acid. Other antioxidants include phenols, bisphenols, polyphenols, hydroquinone derivatives, phenyl phosphites, phenyl thioesters, phenyl amines, phenylene diamines, diphenlamines, tetrakis(methylene 3-(3',5'-di-t-butyl-4'hydroxyphenyl)propionate)methane, tris(2,4-di-tert-butylphenyl)phosphite, 2,4-bis(n-octylthio)-6-(4-hydroxy-3,5-di-tert-butylanilino)-1,3,5-triazine, N',N'-hexamethylene bis (3,5-di-tert-butyl-4-hydroxy-hydrocinnamamide), o,o-di-n-octadecyl-3-5-di-tert-butyl-4-hydroxybenzyl phosphonate, octadecyl 3-(3',5'-di-tert-butyl-4'-hydroxyphenyl) proprionate, thiodiethylene bis-(3,5-di-tert-butyl-4-hydroxy) hydrocinnamate, octylated diphenylamine, 1,3,5-trimethyl-2,4,6-tris(3,5-di-tert-butyl-4-hydroxybenzyl)benzene, 2,6-di-tert-butyl-4-n-butylphenol, 4,4'methylenebis (2,6-di-tert-butylphenol), 2,6-di-tert-butyl-α-dimethylamino-p-cresol, 2,6-di-tert-butyl-4-ethylphenol, combinations of the above, and the like. Additional antioxidants that may be useful in the practice of this invention are listed in: U.S. Patents 3,723,147 (March 1973, assigned to Eastman Kodak); 4,137,201 (January 1979, assigned to Eastman Kodak); 4,839,405 (January 1989, assigned to Plasticolors, Inc.); U.S. published patent application 20030097963 (May 2003, assigned to Eastman Kodak); and WO 200101963, entitled "Method for controlling the flux of penetrants across an adaptable semi-permeable barrier is useful for administering an agent to a mammalian body or a plant and for generating an immune response by vaccinating the mammal", Patent Assignee: Idea AG, Inventors: Cevc, G; Richardson, H; Weiland-Waibel, A.

EXAMPLES

The following examples are illustrative of the present invention and should not be considered as limiting the scope of the invention in any way, as these examples and other equivalents thereof will become apparent to those versed in the art in light of the present disclosure, drawings and accompanying claims.

EXAMPLE 1: POTASSIUM CHLORIDE CORES

Standard cores for use in oral osmotic dosage forms were prepared as follows. First, 5,640 grams of potassium chloride powder were passed through a 45 mesh sieve and dried 23 hours in forced air at 50°C. 300 g of polyvinylpyrrolidone having molecular weight in the range of about 44,000 to 54,000 grams per mole (K2932) was dissolved in 1200 grams of anhydrous ethyl alcohol formula SDA 3A. SDA alcohol refers to specially denatured alcohol formula that comprises 100 parts by volume of ethanol blended with 5 parts by volume of methanol. The dried potassium chloride powder was charged into a Hobart mixing bowl. The ethanol solution of PVP was slowly added to the powders while mixing. Mixing proceeded for about one half hour. The resulting wet mass was passed through a 20-mesh sieve and the resulting noodles were dried in a fume hood at room temperature overnight. The dried noodles were then passed through a 20-mesh sieve yielding 5,097 grams. The resulting granules were transferred to a twin shell mixer. 25.6 grams of magnesium stearate was passed through an 80 mesh sieve over the granules and blended in the mixer for 2 minutes. The final granulation comprised 94.5 wt% potassium chloride, 5 wt% polyvinyl pyrrolidone, 0.5 wt% magnesium stearate.
The resulting granulation was transferred to a Kilian tablet press fitted with 3/8 inch round standard biconcave tooling. The granulation was compressed into tablets with a nominal weight of 532 mg. This equals a nominal potassium chloride content of approximately 500 mg per tablet.

EXAMPLE 2: FORMING TEST SAMPLES

Six membrane formulations (Sample types A-F) were coated onto six separate sublots of osmotic cores from the master batch made according to Example 1. The six batches were spray coated in a Hi-Coater fitted with a 14 inch pan. For each batch the pan was charged with approximately 170 grams of potassium chloride cores and approximately 830 grams of lactose tablets. The lactose tablets provided extra bulk to the bed of tablets to assure proper tumbling action within the pan during the coating process. The semi-permeable membrane-forming compositions were prepared with the ingredients shown in Table 1, by dissolving the ingredients in acetone to make a 5.0 wt % solids solution. Placing the solution container in a warm water bath during this solution preparation step accelerated the dissolution of the components. The membrane-forming compositions were coated onto the cores to provide a semi-permeable membrane for each of the sublots. The coated tablets were then dried overnight in a forced air oven at 40 degrees C.

Table 1

| Sublot | Membrane Weight (Thickness) | CA-398-10 (wt%) | Lutrol F68 (wt%) | Lutrol F127 (wt%) | BHT (wt% of membrane) | BHT (ppm of Lutrol) |
|--------|------------------------------|------------------|-------------------|---------------------|------------------------|----------------------|
| A | 76mg (9.6 mils) | 70.0 | 29.997 | - | 0.003 | 100ppm |
| B | 76 mg (9.6 mils) | 70.0 | 29.982 | - | 0.018 | 600ppm |

(continued)

| Sublot | Membrane Weight (Thickness) | CA-398-10 (wt%) | Lutrol F68 (wt%) | Lutrol F127 (wt%) | BHT (wt% of membrane) | BHT (ppm of Lutrol) |
|---|---|---|---|---|---|---|
| C | 38 mg (5.3 mils) | 77.0 | 22.998 | - | 0.0023 | 100ppm |
| D | 38 mg (5.3 mils) | 77.0 | 22.986 | - | 0.014 | 600ppm |
| E | 39 mg (5.2 mils) | 70.0 | - | 29.997 | 0.003 | 100ppm |
| F | 39 mg (5.2 mils) | 70.0 | - | 29.982 | 0.018 | 600ppm |

CA-398-10 = cellulose acetate with average acetyl content of 39.8 wt%, falling ball viscosity 10 seconds as measured by ASTM test D817 (Formula A).
Lutrol F68 = a:b:a triblock copolymer of ethylene oxide: propylene oxide: ethylene oxide where a = approximately 80, b= approximately 27, and the average molecular weight = 7,680-9,510 grams per mole.
Lutrol F127 = a:b:a triblock copolymer of ethylene oxide: propylene oxide: ethylene oxide where a = approximately 101, b= approximately 56, and the average molecular weight = 9,840-14,600 grams per mole.
BHT = butylated hydroxytoluene.
(Note: The antioxidant content expressed as weight percent is calculated based with respect to the total weight of the dry membrane while the antioxidant content expressed as parts per million is calculated based with respect to the weight of Lutrol.)

Next, a 10 mil exit orifice was mechanically drilled in the coated tablet to provide contact of the core with the exterior of the delivery device.

EXAMPLE 3: AGING AND RELEASE RATE TESTING

The sublots A-F from Example 2 were packaged in 20 cc screw-capped glass vials with 5 systems contained in each vial. The vials of each sublot were then divided into four groups. The resulting groups were then placed in four storage temperatures such that portions of each sublot of vials were stored at 4°C, ambient room temperature, 40°C, and 50°C.

The resulting vials were sampled at storage times 0, 1.5 months, 3 months, 6 months, 12 months, 18 months, and 24 months. At each storage time and temperature, a vial of systems was retrieved and performance was evaluated by measuring the release rate of potassium chloride. Release performance was determined by bagging the finished systems in 40 mesh nylon bags and allowing them to release in a United States Pharmacopeia National Formulary 26/21 Apparatus 7 dissolution tester into test tubes containing de-ionized water thermostated at 37°C. The testing was conducted with 2-hour sampling times over a duration of 26 hours. Release rate of potassium chloride, dm/dt, was measured by conductivity analysis using a conductivity meter and reference solutions of potassium chloride of known concentrations. The average release rate during the zero order rate portion, dm/dt, was determined as the average rate between $\geq 20\%$ and $\leq 70\%$ of cumulative release. Average release rates were then plotted as a function of storage time for a given membrane formulation and for a given storage temperature. The line connecting the data of each plot represents the linear regression of the data. Each plot was also constructed with two horizontal dashed lines which lines represent the average release rate at time zero storage plus 5% and minus 5%, respectively.

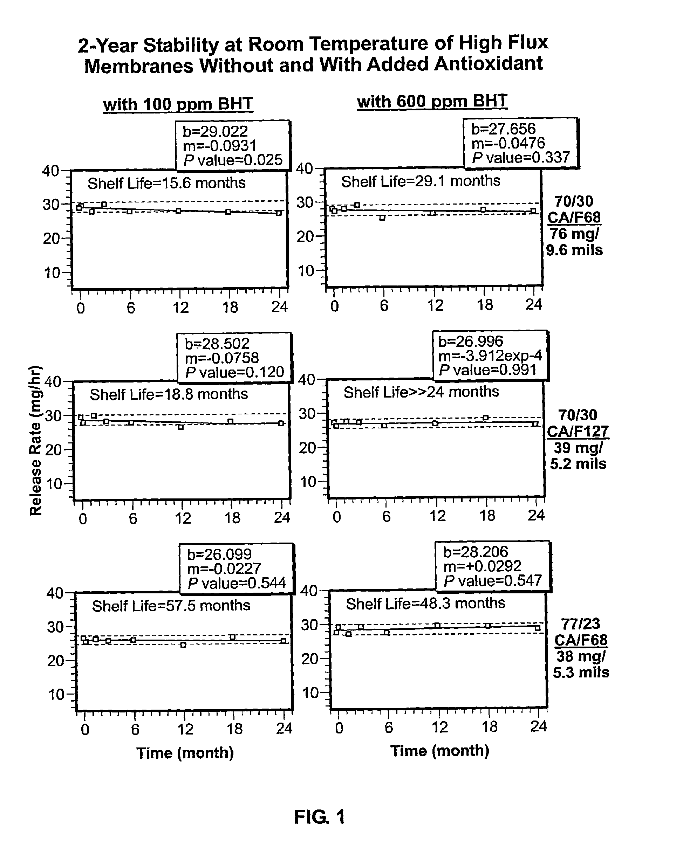

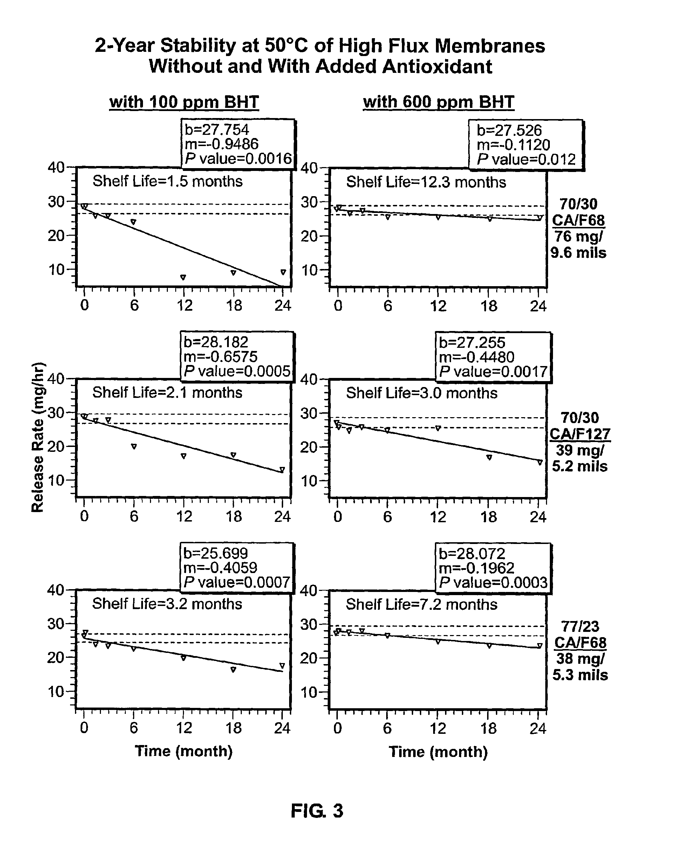

The results of these tests on the systems stored at room temperature, 40°C, and 50°C are shown in Figures 1 to 3, respectively. The systems that had been stored at 4°C showed no changes in stability compared to time zero and therefore are excluded from the analysis of time/temperature effects.

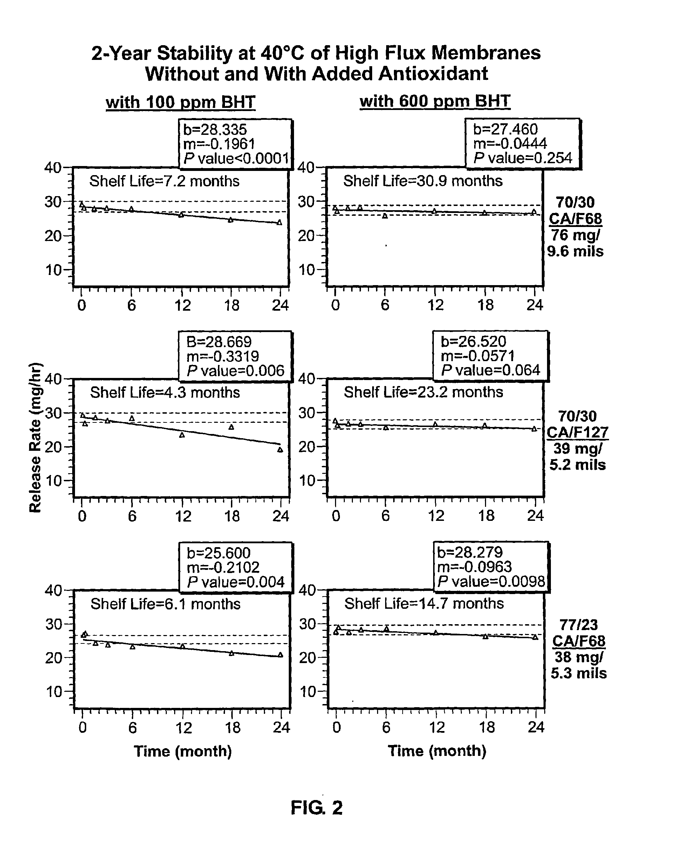

Turning again to Figures 1 to 3, the individual plots located on the left column of each figure represent the performance of membranes stabilized with the standard level of 100 ppm antioxidant. The individual plots located on the right of each figure represent performance of semi-permeable membranes stabilized with 600 ppm antioxidant of the present invention. It is clear from reviewing these plots that the release rate performance of all membranes trend downward with time as represented by the negative slopes, or m-values, of most of the linear regression lines of the individual plots. It is also clear in comparing the plots on the right column to the plots on the left column, that at a given flux enhancer concentration and type, the magnitude of the negative slope is consistently smaller for the membranes with 600 ppm of the present invention compared to the level of 100 ppm antioxidant of the prior art. The smaller slope indicates less change in performance during storage time and therefore better stability over storage time of the present invention compared to the prior art membranes. For example, referring to Figure 2, the membrane weighing 39 mg and formulated with 70 parts cellulose acetate and 30 parts Lutrol F127 formulated with 600 ppm BHT and stored at 40°C has a small negative slope of m = -0.0517. By contrast the equivalent membrane stored in the same condition but formulated with 100 ppm BHT of the prior art has a much larger negative slope of m = -0.3319. The smaller the magnitude of the negative slope, the more functionally stable the membrane. These comparisons clearly illustrate the benefit of the present invention to impart functional stability to these new membranes compared to standard prior art practice.

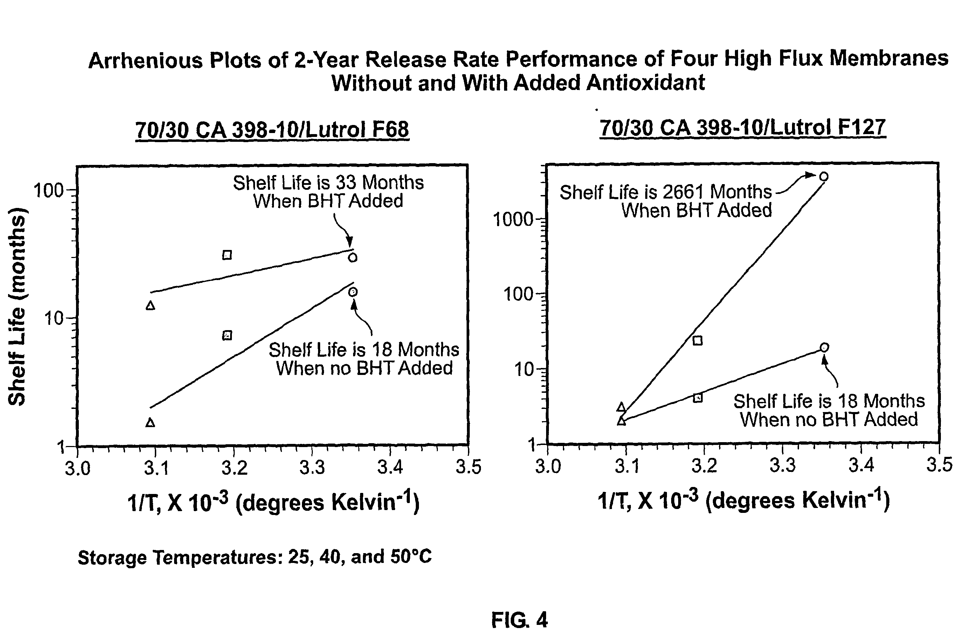

Additional analysis of these data reveal improvements of membrane shelf life stability by practicing the present invention over conventional practice. Figure 4 represents Arrhenious plots of these data. The ordinate of each plot represents the shelf life of a particular membrane composition stored at a particular temperature. The shelf life data were generated as the intersection of the linear regression lines in Figures 1 though 3 with the horizontal dashed line representing a 5% reduction in average release rate compared to the release rate at time zero. Shelf life is plotted as a function of reciprocal storage temperature converted from degrees centigrade to degrees Kelvin. This analysis provides an estimate of long-term shelf life at room temperature of a particular membrane composition. Referring to the plot on the left of Figure 4, the estimated shelf life for a membrane comprising 70 parts cellulose acetate and 30 parts Lutrol F68 with 100 ppm antioxidant is only 18 months. By contrast, the equivalent semi-permeable membrane composition but stabilized with 600 ppm antioxidant of the present invention provides a substantially longer estimated shelf life of 33 months. Likewise, referring to the plot on the right of Figure 4, the estimated shelf life for a membrane comprising 70 parts cellulose acetate and 30 parts Lutrol F127 with 100 ppm antioxidant is only 18 months while the equivalent membrane but stabilized with 600 ppm antioxidant of the present invention provides an estimated shelf life of more than 5 years.

EXAMPLE 4: MEMBRANE TENSILE TESTING

Residual membrane shells from systems that had been stored for one year at 4°C, room temperature, 40°C, or 50°C from Example 3 were retrieved from release rate testing, sliced into two half shells with a razor, and leached in de-ionized water at room temperature with stirring for 5 days to remove the Lutrol flux enhancer. Prior to leaching, the membranes represented 70 parts cellulose acetate with 30 parts Lutrol F68 or 70 parts cellulose acetate with 30 parts Lutrol F127. The membranes had been formulated with either the standard 100 ppm antioxidant of the prior art or the higher antioxidant concentration of the present invention of 600 ppm antioxidant. The resulting leached shells were then dried overnight in a forced air oven at 40°C. Then, the half shells were humidified at 52% relative humidity to constant weight. Finally, the membranes were then re-hydrated in de-ionized water for 4-24 hours prior to tensile testing.

Dogbone samples were punched from the resulting hydrated membrane half shells using a steel rule dogbone die. The die had a neck dimension of 0.125 inch and a length of 0.350 inch. The resulting hydrated dogbone samples were clamped in the jaws and pulled in an Instron Tensile Tester Series IX Automated Materials Testing System. A crosshead speed of 10 mm per minute was used for the measurements. Elongation at break values were measured on at least 3 dogbones for each shell. Average values of elongation at break of the membranes were measured and standard deviations were computed.

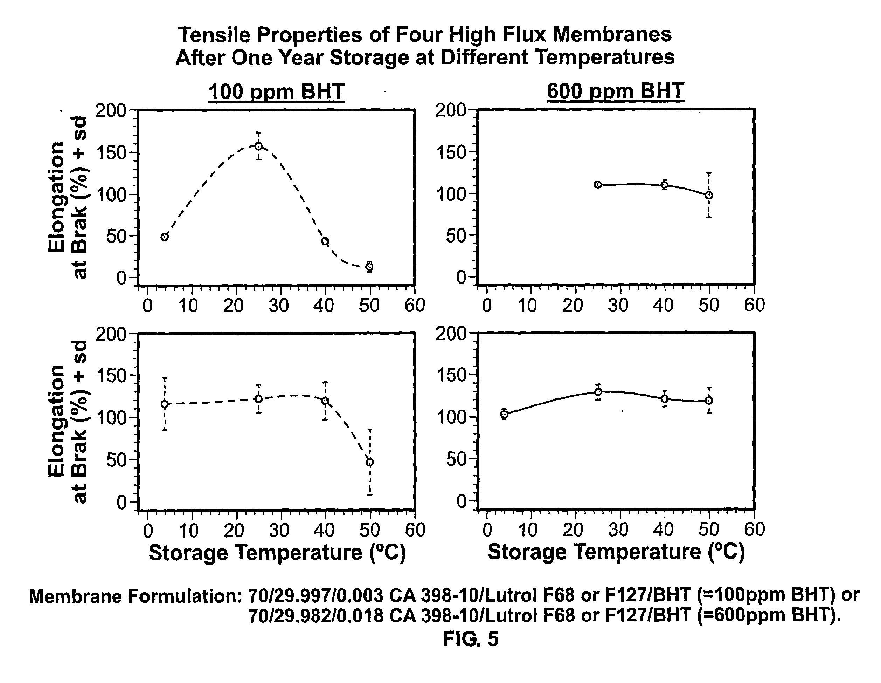

The results of these tensile tests are shown in Figure 5. The upper two frames represent the properties of membranes formulated with 70 parts cellulose acetate and 30 parts Lutrol F68. The lower two frames represent the properties of membranes formulated with 70 parts cellulose acetate and 30 parts Lutrol F127. The two frames on the left represent these membranes formulated with the prior art concentration of 100 ppm antioxidant. The two frames on the right represent these membranes formulated with the present invention concentration of 600 ppm antioxidant. The abscissas of each plot represent the storage temperatures that the membranes had been exposed to during one year of storage.

Referring again to the pair of frames on the left of Figure 5, it is apparent that the membranes of the prior art suffer a degradation of mechanical properties, particularly in the stressed temperature conditions compared to room temperature (i.e. approximately 25°C) storage. The elongation at break value is reduced at 40°C-50°C indicating that the membranes become brittle. By contrast, the pair of frames on the right of Figure 5 illustrate the improved and stable mechanical properties of the present invention membranes as evidenced by no depreciable decline in elongation at break even at 40°C and 50°C storage for a year compared to room temperature 25°C storage.

EXAMPLE 5: ANTIOXIDANT RETENTION

BHT has a relatively low melting point of 70°C and sublimes at elevated temperatures. It is important that an antioxidant be retained within a membrane and not diffuse away if it to present in sufficient quantity over a prolonged storage time to provide a beneficial antioxidant effect. Retention of BHT in membranes stored at elevated temperature was therefore tested.

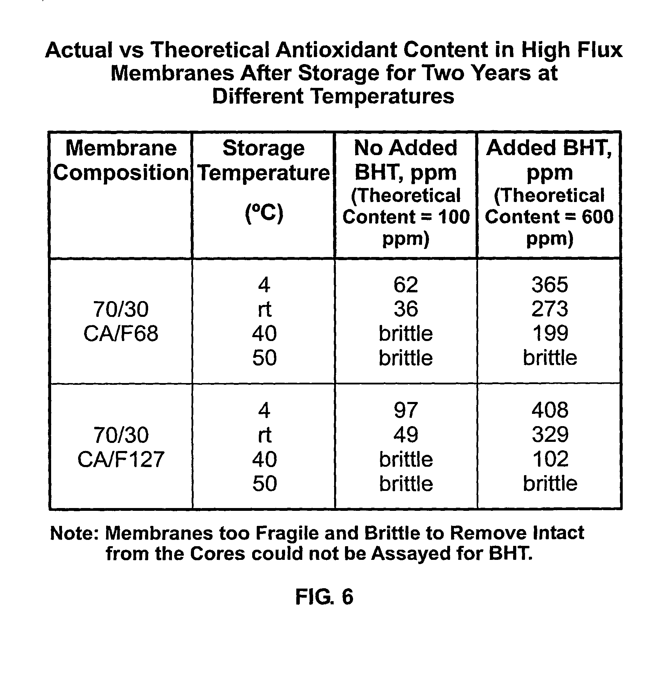

70/30 CA398/Lutrol membranes that had been initially formulated with theoretical concentrations of 100 ppm BHT or 600 ppm BHT and stored for two years at four temperatures in screw-capped glass vials from Example 3 were assayed for BHT content by high performance liquid chromatography. Figure 6 shows the results of these assays. The data reveal that substantial reductions of BHT occur on storage, even at room temperature in sealed vials. Membranes that are initially formulated with higher amounts of BHT of the present invention retain higher amounts during storage and therefore maintain better properties compared to the prior art membranes. Conventional membranes with time zero contents of 100 ppm BHT became brittle and fragile by 2 years storage at 40°C for example whereas those of the present invention formulated with 600 ppm were not brittle or fragile and retained mechanical integrity.

EXAMPLE 6: MOLECULAR WEIGHT TESTING

It is important that the membrane-forming polymer of membranes retain molecular weight as molecular weight is needed to impart good mechanical properties. A reduction in molecular weight can cause membranes that no longer function as needed since they can be come brittle. Samples of cellulose membrane shells that had been retrieved from release testing in Example 3, were leached in water to separate the water soluble components from the water-insoluble cellulose acetate, and dried as described above in Example 4. The resulting dried cellulose acetate membrane shells were then dissolved in tetrahydrofuran. The resulting solutions were passed through a gel permeation chromatograph to determine molecular weight of the polymer. Two membrane samples of each formulation that had been formulated with 30 percent enhancer and that had been stored for two years at 4°C, room temperature, 40°C, and 50°C were characterized.

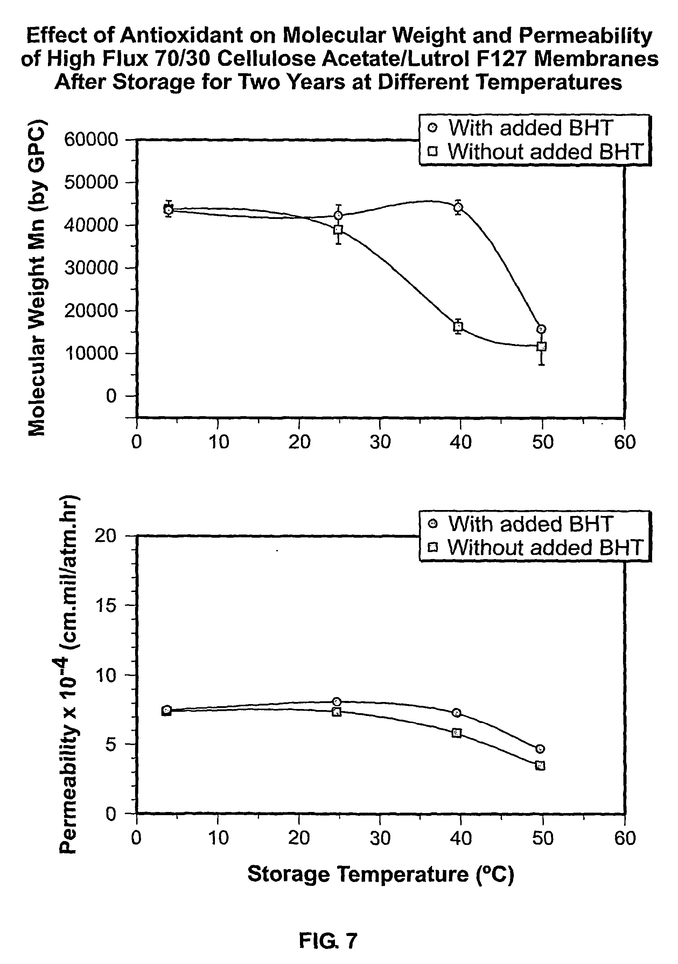

The results of this test on membranes that had originally been formulated with 70 parts cellulose acetate 398-10 and 30 parts Lutrol F127 are shown in the upper frame of Figure 7. Molecular weight of the cellulose acetate blended with Lutrol F127 with the prior art level of 100 ppm antioxidant showed a marked decline in molecular weight with storage temperature after two years of storage at 40 degrees C. This decline is represented by the curve associated with the triangular symbols. By contrast, the molecular weight of the equivalent membrane composition but with 600 ppm antioxidant of the present invention was stable even after storage for 2 years at 40°C compared to the molecular weight of the membrane that had been refrigerated for the same duration of time, as represented by the circular symbols.

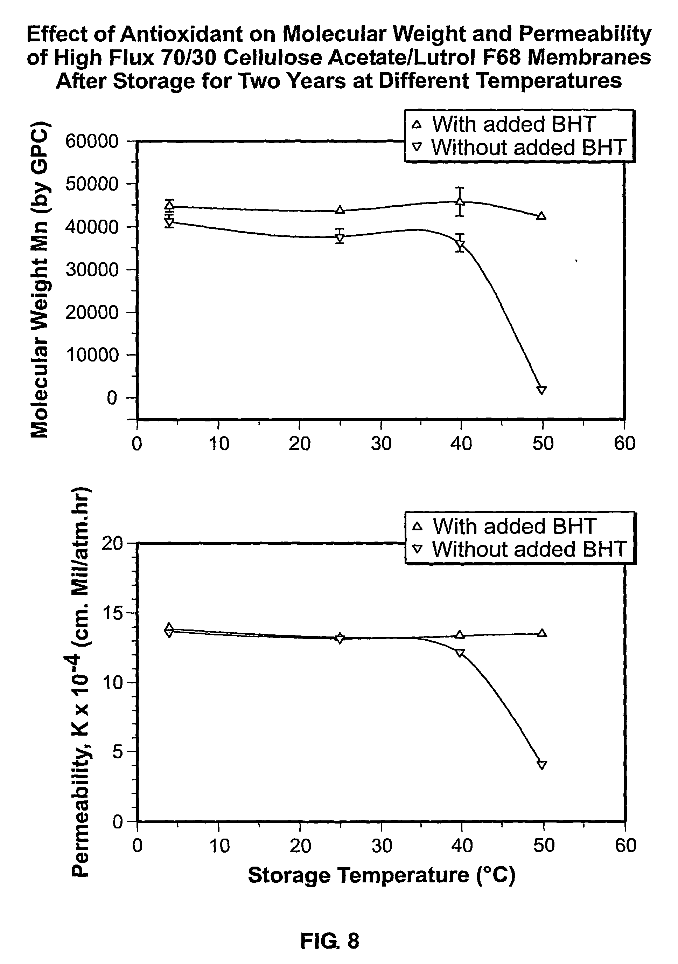

Similarly, the results of this test on membranes that had originally been formulated with 70 parts cellulose acetate 398-10 and 30 parts Lutrol F68 are shown in the upper frame of Figure 8. Molecular weight of the cellulose acetate blended with Lutrol F68 with the prior art level of 100 ppm antioxidant showed a decline in molecular weight with temperature after two years of storage at 50 degree centigrade. This decline is represented by the curve associated with the triangle symbols. By contrast, the molecular weight of the equivalent membrane composition but with 600 ppm antioxidant of the present invention was stable even after storage for 2 years at 40°C to 50°C compared to the molecular weight of the membrane that had been refrigerated for the same duration of time, as represented by the circular symbols.

EXAMPLE 7: PERMEABILITY MEASUREMENTS

Membrane permeability, K, was calculated from Equation 1:

$$K = \frac{(dm/dt)\ h}{\Delta\Pi\ A\ S} \qquad\qquad (Eq\ 1)$$

Where

    h = membrane thickness, mils

    $\Delta\Pi$ = osmotic pressure, atmospheres

    A = membrane area, $cm^2$

    S = drug solubility, mg/ml

    dm/dt = average release rate of drug from the dosage form, mg potassium chloride/hr

The measured values for the tested systems were $\Delta\Pi$ = 245 atmospheres, A = 2.41 $cm^2$, S = 333 mg/ ml.

Thickness of the membrane was measured directly with a table micrometer. Osmotic pressure was measured on saturated solutions of potassium chloride at 37°C using a Knauer model VPO vapor pressure osmometer and standard reference solutions of known osmotic pressure for comparison. Surface area of the tablet core was determined by first measuring the dimensions of the tablet core and then calculating surface area using standard mensuration formulae. Drug solubility was determined by measuring the concentration of a saturated solution of potassium chloride by conductivity analysis using a conductivity meter and reference solutions of potassium chloride of known aqueous concentrations. Average release rate was calculated by measuring the mass of potassium chloride released per 2-hour intervals of time according to the experimental procedures described in Example 3. Then, the total mass of potassium chloride released was summed for all whole 2-hour intervals between 20% and 70% (100 mg to 350 mg of potassium chloride) of the initial dose present within the dosage form (500 mg). Finally, the total mass of drug released during these whole intervals was divided by the sum of these time intervals to arrive at the average release rate. The 20% to 70% cumulative release reference was selected as it represents the period where the dosage form delivers at substantially steady state or zero order rate.

The effect of BHT content in high flux membranes on permeability of the membrane polymer was also evaluated. Referring again to Figure 7, the lower frame shows permeability of 70/30 cellulose acetate 398/Lutrol F127 during storage for two years at four temperatures from the systems in Example 3. The data representing the membrane of the prior art with 100 ppm antioxidant is illustrated in triangular symbols while the data representing the membrane of the present invention with 600 ppm antioxidant is illustrated in circular symbols. Clearly, all membranes show a decline in permeability as storage temperature increases. The data presented illustrate that the membrane of the present invention declines to a lesser extent and are therefore more stable than the prior art membrane as evidenced by the curve for the membranes of the present invention being consistently above the curve for the prior art membrane.

Similarly, the lower frame of Figure 8 shows these parameters for permeability of 70/30 cellulose acetate 398/Lutrol F68 during storage for two years at four temperatures. The data representing the membrane of the prior art is illustrated in triangular symbols while the data representing the membrane of the present invention is illustrated in green circular symbols. Clearly, the membrane of the present invention is more stable as evidenced by the steady value of permeability while by contrast, permeability of the prior art membrane declines precipitously at 40°C to 50°C.

EXAMPLE 8: LIQUID DOSAGE FORM (PROPHETIC)

A liquid osmotic system is prepared that contains a unit dose of ibuprofen. The drug is present as the free acid and the potassium salt dissolved in light mineral oil and contained within a soft gelatin capsule. The soft gelatin capsule is coated with a flexible and impermeable barrier membrane consisting of ethyl acrylate methyl methacrylate 70:30 copolymer. An osmotic push engine is then coated over the flexible impermeable membrane. The osmotic engine consists of 75 parts sodium carboxymethyl cellulose having a molecular weight of 700,000 grams per mole blended with 25 parts sodium chloride. Finally, a high flux membrane comprising 50 parts cellulose acetate 50 parts of Lutrol F127 and 600 parts per million antioxidant ascorbic acid is coated over the osmotic engine. This high flux membrane composition represents on a weight basis 50 parts cellulose acetate, 49.97 parts of Lutrol F127, and 0.03 parts ascorbic acid. A delivery port is drilled through the coated layers but not through the gelatin capsule wall. The resulting system dispenses the unit dose of ibuprofen over a 4-hour period and is stable on shelf storage.

EXAMPLE 9: SOLID DOSAGE FORM (PROPHETIC)

A push pull osmotic system is prepared that contains a 250 mg dose of the analgesic tramadol HCl. A drug granulation of 92 parts tramadol HCl, 5 parts hydroxypropyl cellulose, 2 parts polyvinyl pyrrolidone, and 1 part magnesium stearate is prepared by standard wet granulation procedures using ethanol as the granulating solvent. A push granulation composition is prepared consisting of 55 parts polyoxyethylene 7 million molecular weight, 14 parts hydroxypropyl cellulose 80,000 molecular weight, 29 parts sodium chloride, 1.5 parts ferric oxide, and 0.5 parts magnesium stearate is prepared by standard wet granulation processing. Bilayer tablets are compressed with 13/32 inch round tablet tooling with a drug layer weight of 272 mg and a push layer weight of 137 mg. The bilayer tablets are coated with a 4-mil thick membrane comprising 75 parts cellulose acetate 398-10, 25 parts Lutrol F68, and 700 ppm butylated hydroxyl toluene. On a weight basis, the composition of this membrane represents 75 parts cellulose acetate, 24.9825 parts Lutrol, and .0175 parts BHT. A 15-mil diameter delivery port is drilled across the membrane on the drug layer side. The resulting delivery system dispenses the dose of drug at approximately 50 mg per hour over approximately a 5-hour period. The delivery system is stable when stored on the shelf for a prolonged time.

**Claims**

1.   An oral osmotic dosage form comprising:

a core that comprises a drug layer;
a semi-permeable membrane surrounding the core; and
at least one exit port through the semi-permeable membrane;

wherein the semi-permeable membrane comprises from 23 wt% to 50 wt% of a flux enhancer and from 0.01 wt% to 5 wt% of antioxidant, based on the total dry weight of the semi-permeable membrane.

**2.** The oral osmotic dosage form of claim 1, wherein the oral osmotic dosage form comprises an elementary osmotic pump dosage form.

**3.** The oral osmotic dosage form of claim 1, further comprising a push layer located within the semi-permeable membrane at a point distant from the exit port.

**4.** The oral osmotic dosage form of claim 1, wherein the drug layer comprises a liquid drug formulation.

**5.** The oral osmotic dosage form of claim 1, wherein the antioxidant comprises butylated hydroxyanisol, butylated hydroxytoluene, butylated hydroxymethylphenol, teriary butylhydroquinone, tocopherols, phospholipids, lecithin, editic acid, ascorbic acid, isoascorbic acid, fumaric acid, malic acid, ascorbic acid palmitate, gallic acid, propyl gallate, sodium ascorbate, sodium sulfite, sodium metabisulfite, sodium bisulfite, sodium thiosulfate, sodium formaldehyde sulfoxylate, sulfur dioxide, thioglycerol, thioglycolic acid, cysteine hydrochloride, acetylcysteine, ascorbyl palmitate, hydroquinone, nordihydroguaiaretic acid. Other antioxidants include phenols, bisphenols, polyphenols, hydroquinone derivatives, phenyl phosphites, phenyl thioesters, phenyl amines, phenylene diamines, diphenlamines, tetrakis(methylene 3-(3',5'-di-t-butyl-4'hydroxyphenyl)propionate)methane, tris(2,4-di-tert-butylphenyl)phosphite, 2,4-bis(n-octylthio)-6-(4-hydroxy-3,5-di-tert-butylanilino)-1,3,5-triazine, N',N'-hexamethylene bis (3,5-di-tert-butyl-4-hydroxyhydrocinnamamide), o,o-di-n-octadecyl-3-5-di-tert-butyl-4-hydroxybenzyl phosphonate, octadecyl 3-(3',5'-di-tert-butyl-4'-hydroxyphenyl) proprionate, thiodiethylene bis-(3,5-di-tert-butyl-4-hydroxy) hydrocinnamate, octylated diphenylamine, 1,3,5-trimethyl-2,4,6-tris(3,5-di-tert-butyl-4-hydroxybenzyl)benzene, 2,6-di-tert-butyl-4-n-butylphenol, 4,4'methylenebis (2,6-di-tert-butylphenol), 2,6-di-tert-butyl-$\alpha$-dimethylamino-p-cresol, 2,6-di-tert-butyl-4-ethylphenol, or combinations of the above.

**6.** The oral osmotic dosage form of claim 1, wherein the semi-permeable membrane comprises from 0.01 wt% to 3 wt% of the antioxidant, based on the total dry weight of the semi-permeable membrane.

**7.** The oral osmotic dosage form of claim 6, wherein the semi-permeable membrane comprises from 0.01 wt % to 1 wt % of the antioxidant, based on the total dry weight of the semi-permeable membrane.

**8.** A method of making an oral osmotic dosage form comprising:

providing a semi-permeable membrane that comprises from 23 wt% to 50 wt% of a flux enhancer and from 0.01 wt% to 5 wt% of antioxidant, based on the total dry weight of the semi-permeable membrane;
coating the semi-permeable membrane on a core that comprises a drug layer; and
forming at least one exit port through the semipermeable membrane.

**9.** The method of claim 8, wherein the oral osmotic dosage form comprises an elementary osmotic pump dosage form.

**10.** The method of claim 8, wherein the core further comprises a push layer.

**11.** The method of claim 8, wherein the drug layer comprises a liquid drug formulation located within the semi-permeable membrane at a point distant from the exit port.

**12.** The method of claim 8, wherein the antioxidant comprises butylated hydroxyanisol, butylated hydroxytoluene, butylated hydroxymethylphenol, teriary butylhydroquinone, tocopherols, phospholipids, lecithin, editic acid, ascorbic acid, isoascorbic acid, fumaric acid, malic acid, ascorbic acid palmitate, gallic acid, propyl gallate, sodium ascorbate, sodium sulfite, sodium metabisulfite, sodium bisulfite, sodium thiosulfate, sodium formaldehyde sulfoxylate, sulfur dioxide, thioglycerol, thioglycolic acid, cysteine hydrochloride, acetylcysteine, ascorbyl palmitate, hydroquinone, nordihydroguaiaretic acid. Other antioxidants include phenols, bisphenols, polyphenols, hydroquinone derivatives, phenyl phosphites, phenyl thioesters, phenyl amines, phenylene diamines, diphenlamines, tetrakis(methylene 3-(3',5'-di-t-butyl-4'hydroxyphenyl)propionate)methane, tris(2,4-di-tert-butylphenyl)phosphite, 2,4-bis(n-octylthio)-6-(4-hydroxy-3,5-di-tert-butylani)ino)-1,3,5-triazine, N',N'-hexamethylene bis (3,5-di-tert-butyl-4-hydroxy-hydrocinnamamide), o,o-di-n-octadecyl-3-5-di-tert-butyl-4-hydroxybenzyl phosphonate, octadecyl 3-(3',5'-di-tert-butyl-4'-hydroxyphenyl) proprionate, thiodiethylene bis-(3,5-di-tert-butyl-4-hydroxy) hydrocinnamate, octylated diphenylamine, 1,3,5-trimethyl-2,4,6-tris(3,5-di-tert-butyl-4-hydroxybenzyl)benzene, 2,6-di-tert-butyl-4-n-butylphenol, 4,4'methylenebis (2,6-di-tert-butylphenol), 2,6-di-tert-butyl-$\alpha$-dimethylamino-p-cresol, 2,6-di-tert-butyl-4-ethylphenol, or combinations of the above.

13. The method of claim 8, wherein the semi-permeable membrane comprises from 0.01 wt% to 3 wt% of the antioxidant, based on the total dry weight of the semi-permeable membrane.

14. The method of claim 13, wherein the semi-permeable membrane comprises from 0.01 wt % to 1 wt % of the antioxidant, based on the total dry weight of the semi-permeable membrane.

15. An oral osmotic dosage form made according to the method of claim 8.


**Patentansprüche**

1. Orale osmotische Darreichungsform umfassend:

einen Kern, der eine Arzneimittelschicht aufweist;
eine semipermeable Membran, die den Kern umschließt; und
mindestens eine Ausgangsöffnung durch die semipermeable Membran;

wobei die semipermeable Membran von 23 Gew.% bis 50 Gew.-% eines Flussverstärkers und von 0,01 Gew.-% bis 5 Gew.-% eines Antioxidationsmittels, basierend auf dem gesamten Trockengewicht der semipermeablen Membran, umfasst.

2. Orale osmotische Darreichungsform, bei der die osmotische Darreichungsform eine elementare osmotische Pumpdarreichungsform umfasst.

3. Orale osmotische Darreichungsform nach Anspruch 1, die des weiteren eine Schubschicht umfasst, die innerhalb der semipermeablen Membran an einem Punkt entfernt von der Ausgangsöffnung lokalisiert ist.

4. Orale osmotische Darreichungsform nach Anspruch 1, bei der die Arzneimittelschicht eine flüssige Arzneimittelformulierung umfasst.

5. Orale osmotische Darreichungsform nach Anspruch 1, bei der das Antioxidationsmittel umfasst Butylhydroxyanisol, Butylhydroxytoluol, Butylhydroxymethylphenol, tertiäres Butylhydrochinon, Tocopherole, Phospholipide, Lecithin, Edetinsäure, Ascorbinsäure, Isoascorbinsäure, Fumarsäure, Äpfelsäure, Ascorbinsäurepalmitat, Gallussäure, Propylgallat, Natriumascorbat, Natriumsulfit, Natriummetabisulfit, Natriumbisulfit, Natriumthiosulfat, Natriumformaldehydsulfoxylat, Schwefeldioxid, Thioglycerol, Thioglykolsäure, Cysteinhydrochlorid, Acetylcystein, Ascorbylpalmitat, Hydrochionon, Nordihydrogujaretsäure, andere Antioxidationsmittel schließen Phenole, Biphenole, Polyphenole, Hydrochinonderivate, Phenylphosphite, Phenylthioester, Phenylamine, Phenylendiamine, Diphenylamine, tetra-kis (Methylen-3-(3',5'-di-t-butyl-4'-hydroxyphenyl)propionat)methan, tris(2,4-di-tert-Butylphenyl)phosphit, 2,4-bis(n-Octylthio)-6-(4-hydroxy-3,5-di-tert-butyl-anilino)-1,3,5-triazin, N',N'-Hexamethylen-bis(3,5-di-tert-butyl-4-hydroxyhydrocinnanmamid), o,o-di-n-Octadecyl-3-5-di-tert-butyl-4-hydroxybenzyl-phosphonat, Octadecyl-3-(3',5'-di-tert-butyl-4'-hydroxyphenyl)propinonat, Thiodiethylen-bis-(3,5-di-tert-butyl-4-hydroxy)hydrocinnamat, octyliertes Diphenylamin, 1,3,5-Trimethyl-2,4,6-tris(3,5-di-tert-butyl-4-hydroxybenzyl)-benzol, 2,6-di-tert-Butyl-4-n-butylphenol,4,4'-Methylen-bis-(2,6-di-tert-butylphenol), 2,6-di-tert-Butyl-$\alpha$-dimethylamino-p-cresol, 2,6-di-tert-Butyl-4-ethylphenol oder Kombinationen der obigen, ein.

6. Orale osmotische Darreichungsform nach Anspruch 1, bei der die semipermeable Membran von 0,01 Gew.-% bis 3 Gew.-% Antioxidationsmittel, basierend auf dem gesamten Trockengewicht der semipermeablen Membran, umfasst.

7. Orale osmotische Darreichungsform nach Anspruch 1, bei der die semipermeable Membran von 0,01 Gew.-% bis 1 Gew.-% Antioxidationsmittel, basierend auf dem gesamten Trockengewicht der semipermeablen Membran, umfasst.

8. Verfahren zur Herstellung einer oralen osmotischen Darreichungsform umfassend:

Bereitstellen einer semipermeablen Membran, die von 23 Gew.-% bis 50 Gew.-% eines Flussverstärkers und von 0,01 Gew.-% bis 5 Gew.-% eines Antioxidationsmittels, basierend auf dem gesamten Trockengewicht der semipermeablen Membran, umfasst;
Auftragen der semipermeablen Membran auf einen Kern, der eine Arzneimittelschicht umfasst; und
Bilden von mindestens einer Ausgangsöffnung durch die semipermeable Membran.

9. Verfahren nach Anspruch 8, bei der die orale osmotische Darreichungsform eine elementare osmotische Pumpdarreichungsform umfasst.

10. Verfahren nach Anspruch 8, bei der der Kern eine Schubschicht umfasst.

11. Verfahren nach Anspruch 8, bei der Arzneimittelschicht eine flüssige Arzneimittelformulierung umfasst, die innerhalb der semipermeablen Membran an einem Punkt entfernt von der Ausgangsöffnung lokalisiert ist.

12. Verfahren nach Anspruch 8, bei dem das Antioxidationsmittel umfaßt Butylhydroxyanisol, Butylhydroxytoluol, Butylhydroxymethylphenol, tertiäres Butylhydrochinon, Tocopherole, Phospholipide, Lecithin, Edetinsäure, Ascorbinsäure, Isoascorbinsäure, Fumarsäure, Äpfelsäure, Ascorbinsäurepalmitat, Gallussäure, Propylgallat, Natriumascorbat, Natriumsulfit, Natriummetabisulfit, Natriumbisulfit, Natriumthiosulfat, Natriumformaldehydsulfoxylat, Schwefeldioxid, Thioglycerol, Thioglykolsäure, Cysteinhydrochlorid, Acetylcystein, Ascorbylpalmitat, Hydrochionon, Nordihydrogujaretsäure, andere Antioxidationsmittel schließen Phenole, Biphenole, Polyphenole, Hydrochinonderivate, Phenylphosphite, Phenylthioester, Phenylamine, Phenylendiamine, Diphenylamine, tetra-kis(Methylen-3-(3',5'-di-t-butyl-4'hydroxyphenyl)propionat)methan, tris(2,4-di-tert-Butylphenyl)phosphit, 2,4-bis(n-Octylthio)-6-(4-hydroxy-3,5-di-tert-butylanilino)-1,3,5-triazin, N',N'-Hexamethylen-bis(3,5-di-tert-butyl-4-hydroxy-hydrocinnanmamid), o,o-di-n-Octadecyl-3-5-di-tert-butyl-4-hydroxybenzyl-phosphonat, Octadecyl-3-(3',5'-di-tert-butyl-4'-hydroxyphenyl) propionat, Thiodiethylen-bis-(3,5-di-tert-butyl-4-hydroxy)hydrocinnamat, octyliertes Diphenylamin, 1,3,5-Trimethyl-2,4,6-tris(3,5-di-tert-butyl-4-hydroxybenzyl)-benzol, 2,6-di-tert-Butyl-4-n-butylphenol, 4,4'-Methylen-bis-(2,6-di-tert-butylphenol), 2,6-di-tert-Butyl-$\alpha$-dimethylamino-p-cresol, 2,6-di-tert-Butyl-4-ethylphenol oder Kombinationen der obigen, ein.

13. Verfahren nach Anspruch 8, bei dem die semipermeable Membran von 0,01 Gew.-% bis 3 Gew.-% Antioxidationsmittel, basierend auf dem gesamten Trockengewicht der semipermeablen Membran, umfasst.

14. Verfahren nach Anspruch 13, bei dem die semipermeable Membran von 0,01 Gew.-% bis 1 Gew.-% Antioxidationsmittel, basierend auf dem gesamten Trockengewicht der semipermeablen Membran, umfasst.

15. Orale osmotische Darreichungsform, die in Übereinstimmung mit dem Verfahren nach Anspruch 8 hergestellt ist.


**Revendications**

1. Forme posologique osmotique orale comprenant :

- un coeur qui comprend une couche de médicament ;
- une membrane semi-perméable entourant le coeur ; et
- au moins un orifice de sortie à travers la membrane semi-perméable ;

la membrane semi-perméable comprenant de 23 % en poids à 50 % en poids d'un agent augmentant le flux et de 0,01 % en poids à 5 % en poids d'un anti-oxydant, sur la base du poids sec total de la membrane semi-perméable.

2. Forme posologique osmotique orale selon la revendication 1, dans laquelle la forme posologique osmotique orale comprend une forme posologique de pompe osmotique élémentaire.

3. Forme posologique osmotique orale selon la revendication 1, comprenant en outre une couche de poussée située à l'intérieur de la membrane semi-perméable à un point distant de l'orifice de sortie.

4. Forme posologique osmotique orale selon la revendication 1, dans laquelle la couche de médicament comprend une formulation de médicament liquide.

5. Forme posologique osmotique orale selon la revendication 1, dans laquelle l'anti-oxydant comprend l'hydroxyanisol butylé, l'hydroxytoluène butylé, l'hydroxyméthyl phénol butylé, la tert.-butylhydroquinone, les tocophérols, les phospholipides, la lécithine, l'acide éditique, l'acide ascorbique, l'acide isoascorbique, l'acide fumarique, l'acide malique, l'acide ascorbique palmitate, l'acide gallique, le gallate de propyle, l'ascorbate de sodium, le sulfite de sodium, le métabisulfite de sodium, le bisulfite de sodium, le thiosulfate de sodium, le formaldéhyde sulfoxylate de sodium, le dioxyde de soufre, le thioglycérol, l'acide thioglycolique, le chlorhydrate de cystéine, l'acétylcystéine, le palmitate d'ascorbyle, l'hydroquinone, l'acide nordhydrogualarétique, d'autres anti-oxydants comprenant les phénols, les bisphénols, les polyphénols, les dérivés d'hydroquinone, les phényl phosphites, les phényl thioesters, les phényl amines, les phénylène diamines, les diphénylamines, le tétrakis(méthylène 3-(3' 5'-di-t-butyl-4'-hydroxyphényl)propionate) méthane, le tris(2,4-di-tert.-butylphényl)phosphite, le 2,4-bis(n-octylthio)-6-(4-hydroxy-3,5-di-tert.-butylanilino)-1,3,5-triazine, le N, N'-hexaméthylène bis(3,5-di-tert.-butyl-4-hydroxy-hydrocinnamamide), le phosphonate de O, O-di-n-octadécyl-3,5-di-tert.-butyl-4-hydroxybenzyl phosphonate, le 3-(3', 5'-di-tert.-butyl-4'-hydroxyphényl)propionate d'octadécyle, le thiodiéthylène bis(-3,5-di-tert.-butyl-4-hydroxy)hydrocinnamate, la diphénylamine octylée, le 1,3,5-triméthyl-2,4,6-tris(3,5-di-tert.-butyl-4-hydroxybenzyl)benzène, le 2,6-di-tert.-butyl-4-n-butylphénol, le 4,4' méthylène bis(2,6-di-tert.-butylphénol), le 2,6-di-tert.-butyl-$\alpha$-diméthylamino-p-crésol, le 2,6-di-tert.-butyl-4-éthylphénol ou des combinaisons des anti-oxydants ci-dessus.

6. Forme posologique osmotique orale selon la revendication 1, dans laquelle la membrane semi-perméable comprend de 0,01 % en poids à 3 % en poids de l'anti-oxydant sur la base du poids total sec de la membrane semi-perméable.

7. Forme posologique osmotique orale selon la revendication 1, dans laquelle la membrane semi-perméable comprend de 0,01 % en poids à 1 % en poids de l'anti-oxydant sur la base du poids sec total de la membrane semi-perméable.

8. Procédé de fabrication d'une forme posologique osmotique orale comprenant les opérations consistant à :

   - prendre une membrane semi-perméable qui comprend de 23 % en poids à 50 % en poids d'un agent augmentant le flux et de 0,01 % en poids à 5 % en poids d'un anti-oxydant, sur la base du poids sec total de la membrane semi-perméable ;
   - appliquer en revêtement la membrane semi-perméable sur un noyau qui comprend une couche de médicament ; et
   - former au moins un orifice de sortie à travers la membrane semi-perméable.

9. Procédé selon la revendication 8, dans lequel la forme posologique osmotique orale comprend une forme posologique de pompe osmotique élémentaire.

10. Procédé selon la revendication 8, dans lequel le coeur comprend en outre une couche de poussée.

11. Procédé selon la revendication 8, dans lequel la couche de médicament comprend une formulation de médicament liquide située à l'intérieur de la membrane semi-perméable à un point distant de l'orifice de sortie.

12. Procédé selon la revendication 8, dans lequel l'anti-oxydant comprend l'hydroxyanisol butylé, l'hydroxytoluène butylé, l'hydroxyméthyl phénol butylé, la tert.-butylhydroquinone, les tocophérols, les phospholipides, la lécithine, l'acide éditique, l'acide ascorbique, l'acide isoascorbique, l'acide fumarique, l'acide malique, l'acide ascorbique palmitate, l'acide gallique, le gallate de propyle, l'ascorbate de sodium, le sulfite de sodium, le métabisulfite de sodium, le bisulfite de sodium, le thiosulfate de sodium, le formaldéhyde sulfoxylate de sodium, le dioxyde de soufre, le thioglycérol, l'acide thioglycolique, le chlorhydrate de cystéine, l'acétylcystéine, le palmitate d'ascorbyle, l'hydroquinone, l'acide nordhydrogualarétique, d'autres anti-oxydants comprenant les phénols, les bisphénols, les polyphénols, les dérivés d'hydroquinone, les phényl phosphites, les phényl thioesters, les phényl amines, les phénylène diamines, les diphénylamines, le tétrakis(méthylène 3-(3' 5'-di-t-butyl-4'-hydroxyphényl)propionate)méthane, le tris(2,4-di-tert.-butylphényl)phosphite, le 2,4-bis(n-octylthio)-6-(4-hydroxy-3,5-di-tert.-butylanilino)-1,3,5-triazine, le N, N'-hexaméthylène bis(3,5-di-tert.-butyl-4-hydroxyhydrocinnamamide), le phosphonate de O, O-di-n-octadécyl-3,5-di-tert.-butyl-4-hydroxybenzyl phosphonate, le 3-(3', 5'-di-tert.-butyl-4'-hydroxyphényl)propionate d'octadécyle, le thiodiéthylène bis(-3,5-di-tert.-butyl-4-hydroxy)hydrocinnamate, la diphénylamine octylée, le 1,3,5-triméthyl-2,4,6-tris(3,5-di-tert.-butyl-4-hydroxybenzyl)benzène, le 2,6-di-tert.-butyl-4-n-butylphénol, le 4,4' méthylène bis(2,6-di-tert.-butylphénol), le 2,6-di-tert.-butyl-$\alpha$-diméthylamino-p-crésol, le 2,6-di-tert.-butyl-4-éthylphénol ou des combinaisons des anti-oxydants ci-dessus.

13. Procédé selon la revendication 8, dans lequel la membrane semi-perméable comprend de 0,01 % en poids à 3 % en poids de l'anti-oxydant, sur la base du poids sec total de la membrane semi-perméable.

14. Procédé selon la revendication 13, dans lequel la membrane semi-perméable comprend 0,01 % en poids à 1 % en poids de l'anti-oxydant, sur la base du poids sec total de la membrane semi-perméable.

15. Forme posologique osmotique orale fabriquée par le procédé tel que défini à la revendication 8.

## 2-Year Stability at Room Temperature of High Flux
## Membranes Without and With Added Antioxidant

FIG. 1

**2-Year Stability at 40°C of High Flux Membranes
Without and With Added Antioxidant**

FIG. 2

## 2-Year Stability at 50°C of High Flux Membranes
## Without and With Added Antioxidant

FIG. 3

# Arrhenious Plots of 2-Year Release Rate Performance of Four High Flux Membranes Without and With Added Antioxidant

### 70/30 CA 398-10/Lutrol F68

Shelf Life is 33 Months When BHT Added

Shelf Life is 18 Months When no BHT Added

### 70/30 CA 398-10/Lutrol F127

Shelf Life is 2661 Months When BHT Added

Shelf Life is 18 Months When no BHT Added

Storage Temperatures: 25, 40, and 50°C

FIG. 4

Tensile Properties of Four High Flux Membranes
After One Year Storage at Different Temperatures

**100 ppm BHT**

**600 ppm BHT**

Elongation at Brak (%) + sd

Storage Temperature (°C)

Membrane Formulation: 70/29.997/0.003 CA 398-10/Lutrol F68 or F127/BHT (=100ppm BHT) or 70/29.982/0.018 CA 398-10/Lutrol F68 or F127/BHT (=600ppm BHT).

FIG. 5

## Actual vs Theoretical Antioxidant Content in High Flux Membranes After Storage for Two Years at Different Temperatures

| Membrane Composition | Storage Temperature (°C) | No Added BHT, ppm (Theoretical Content = 100 ppm) | Added BHT, ppm (Theoretical Content = 600 ppm) |
|---|---|---|---|
| 70/30 CA/F68 | 4 rt 40 50 | 62 36 brittle brittle | 365 273 199 brittle |
| 70/30 CA/F127 | 4 rt 40 50 | 97 49 brittle brittle | 408 329 102 brittle |

Note: Membranes too Fragile and Brittle to Remove Intact from the Cores could not be Assayed for BHT.

## FIG. 6

**Effect of Antioxidant on Molecular Weight and Permeability of High Flux 70/30 Cellulose Acetate/Lutrol F127 Membranes After Storage for Two Years at Different Temperatures**

**FIG. 7**

**Effect of Antioxidant on Molecular Weight and Permeability
of High Flux 70/30 Cellulose Acetate/Lutrol F68 Membranes
After Storage for Two Years at Different Temperatures**

FIG. 8

**FIG. 9**

**FIG. 10**

20 mil Orifice

Liquid Formulation

Semi-Permeable Membrane

HPMC Capsule

Barrier Layer

Push Layer:
Generic Polyox 7000K

**FIG. 11**

Delivery Orifice

Rate-Controlling Membrane

Soft Gelatin Capsule

Osmotic-Layer

Barrier Layer

Liquid Drug Formulation

**FIG. 12**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3845770 A
- US 3916899 A
- US 3995631 A
- US 4008719 A
- US 4111202 A
- US 4160020 A
- US 4327725 A
- US 4519801 A
- US 4578075 A
- US 4681583 A
- US 5019397 A
- US 5156850 A
- US 2004115262 A
- US 4200098 A
- US 4612008 A
- US 5082668 A
- US 5091190 A
- US 2707154 A
- US 3573936 A
- US 3637772 A

- US 4038434 A
- US 4186465 A
- US 4559237 A
- US 6174547 B
- US 6419952 B
- US 6596314 B
- US 5324280 A
- US 4077407 A
- US 3173876 A
- US 3276586 A
- US 3541005 A
- US 3541006 A
- US 3546142 A
- US 3133132 A
- US 3723147 A
- US 4137201 A
- US 4839405 A
- US 20030097963 A
- WO 200101963 A

**Non-patent literature cited in the description**

- **SANTUS ; BAKER.** Osmotic drug delivery: a review of the patent literature. *Journal of Controlled Release,* 1995, vol. 35, 1-21
- Encyclopedia of Polymer Science and Technology. Interscience Publishers, Inc, 1964, vol. 3, 325-354

- **SCOTT, J. R. ; ROFF, W. J.** Handbook of Common Polymers. CRC Press, 1971
- McCutheon's Detergents and Emulsifiers. Annual and BASF Pluronic and Tetronic Surfactants. 1979